(19) Europäisches Patentamt
European Patent Office
Office européen des brevets

(11) Veröffentlichungsnummer: **0 346 709 B1**

(12) **EUROPÄISCHE PATENTSCHRIFT**

(45) Veröffentlichungstag der Patentschrift: **19.11.92**

(51) Int. Cl.5: **A01N 37/46**, A01N 53/00

(21) Anmeldenummer: **89110094.3**

(22) Anmeldetag: **03.06.89**

# (54) Verwendung von acylierten Aminosäureester-Derivaten zur Insekten- und Milbenabwehr.

(30) Priorität: **16.06.88 DE 3820528**

(43) Veröffentlichungstag der Anmeldung:
**20.12.89 Patentblatt 89/51**

(45) Bekanntmachung des Hinweises auf die Patenterteilung:
**19.11.92 Patentblatt 92/47**

(84) Benannte Vertragsstaaten:
**AT BE CH DE ES FR GB GR IT LI NL SE**

(56) Entgegenhaltungen:
**EP-A- 0 136 615**
**EP-A- 0 221 502**
**US-A- 4 127 672**

(73) Patentinhaber: **BAYER AG**
**W-5090 Leverkusen 1 Bayerwerk(DE)**

(72) Erfinder: **Krüger, Bernd-Wieland, Dr.**
**Unterboschbach 19**
**W-5060 Bergisch-Gladbach 2(DE)**
Erfinder: **Sasse, Klaus, Dr.**
**Puetzweg 13**
**W-5060 Bergisch-Gladbach 2(DE)**
Erfinder: **Lunkenheimer, Winfried, Dr.**
**Bismarckstrasse 29**
**W-5600 Wuppertal 1(DE)**
Erfinder: **Hoever, Franz-Peter, Dr.**
**Kunstfelder Strasse 25**
**W-5000 Köln 80(DE)**
Erfinder: **Nentwig, Günther, Dr.**
**Haferkamp 10**
**W-5000 Köln 80(DE)**
Erfinder: **Behrenz, Wolfgang, Dr.**
**Untergruendemich 14**
**W-5063 Overath(DE)**

EP 0 346 709 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von teilweise bekannten acylierten α-Aminosäureester-Derivaten zur Insekten- und Milbenabwehr.

Mittel, die Insekten und Milben abweisen (Repellents), haben die Aufgabe, schädliche oder lästige Gliederfüßler von Berührung sowie vom Stechen und Saugen oder Beißen an für sie anlockenden Oberflächen, etwa der Haut von Tieren und Menschen abzuhalten, wenn diese zuvor mit solchen Mitteln behandelt wurden.

Als Repellents wurden bereits zahlreiche Wirkstoffe vorgeschlagen. (Vergl. z.B. K.H.Büchel in Chemie der Pflanzenschutz- und Schädlingsbekämpfungsmittel; Herausgeber: R. Wegler, Bd. 1, Springer Verlag Berlin, Heidelberg, New York, 1970 S. 487 ff).

Besonders bekannt und seit längerer Zeit in Gebrauch sind 3-Methyl-benzoesäurediethylamid (DEET), Dimethylphthalat und 2-Ethyl-hexandiol-1,3, von denen vor allem das DEET in der Praxis eine erhebliche Bedeutung erlangt hat [siehe z.B. R.K.Kocher, R.S. Dixit, C.I. Somaya; Indian J. Med. Res. 62, 1 (1974)].

Ein erheblicher Nachteil der bekannten Repellents ist ihre zum Teil relativ kurze (nur wenige Stunden) anhaltende Dauerwirkung.

Ein Teil der durch die nachfolgende Formel (I) definierten Verbindungen ist bekannt (vgl. z.B. EP- A 136 615 und EP-A 221 502).

Die Publikation EP-A 136 615 bezieht sich auf 1-Methylaminocyclopropan-1-carbonsäure-Derivate sowie deren pflanzenverträgliche Säureadditionssalze und ihre Verwendung zur Regulierung des Pflanzenschutzwachstums.

EP-A 221 502 betrifft substituierte 1-Amino-cyclopropancarbonsäure-Derivate und ihre Verwendung als Schädlingsbekämpfungsmittel, insbesondere als Fungizide.

Eine insekten- und milbenabweisende Wirkung dieser Verbindungen ist jedoch bisher nicht bekannt geworden.

Es wurde nun gefunden, daß die teilweise bekannten acylierten α-Aminosäureester-Derivate der Formel

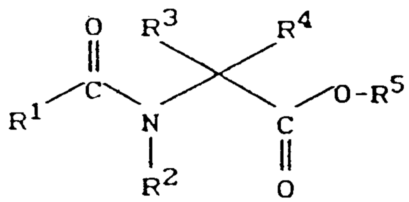

(I)

in welcher

- $R^1$ für Wasserstoff oder für einen gegebenenfalls substituierten Alkyl-, Alkenyl- oder Alkinylrest steht,
- $R^2$ und $R^5$ gleich oder verschieden sind und für gegebenenfalls substituierte Alkyl-, Alkenyl- oder Alkinylreste stehen und
- $R^3$ und $R^4$ für Wasserstoff, gegebenenfalls substituiertes Alkyl, Aralkyl oder Aryl stehen oder gemeinsam mit dem Atom, an welches sie gebunden sind, einen gegebenenfalls substituierten, monocyclischen Ring bilden,

eine starke insekten- und milbenabweisende Wirkung (Repellentwirkung) besitzen.

Die Repellentwirkung ist erheblich besser als die der aus dem Stand der Technik bekannten Repellents. Die erfindungsgemäßen Wirkstoffe stellen somit eine wertvolle Bereicherung der Technik dar.

Die vorliegende Erfindung betrifft somit die Verwendung acylierter α-Aminosäureester-Derivate der allgemeinen Formel (I) zur Insekten- und Milbenabwehr.

Die daraus hergestellten insekten- und milbenabweisenden Mittel sind gekennzeichnet durch den Gehalt an mindestens einem acylierten α-Aminosäureester-Derivat der allgemeinen Formel (I).

Die insekten- und milbenabweisenden Mittel, die mindestens ein Derivat der Formel (I) enthalten, können auch weitere Insektenabwehrmittel enthalten. Hier kommen alle praktisch üblichen Repellentien in Frage (Vergl. z.B. K.H.Büchel in Chemie der Pflanzenschutz- und Schädlingsbekämpfungsmittel; Herausgeber: R.Wegler, Bd. 1, Springer Verlag Berlin, Heidelberg, New York, 1970 S. 487 ff).

Im Falle der Repellentkombinationen werden bevorzugt die acylierten α-Aminosäureester der allgemeinen Formel (I) zusammen mit repellenten Carbonsäureamiden, 1,3-Alkandiolen und Carbonsäureestern verwendet. Im einzelnen seien genannt: 3-Methyl-benzoesäurediethylamid (DEET), 2-Ethyl-hexandiol-1,3 (Rutgers 612) und Phthalsäuredimethylester.

Die erfindungsgemäß verwendbaren acylierten α-Aminosäureester-Derivate sind durch die allgemeine Formel (I) charakterisiert.

Die in der Formel (I) angegebenen Reste haben vorzugsweise die folgende Bedeutung:

Die gegebenenfalls substituierte Alkylgruppe in den Resten $R^1$ und $R^5$ ist geradkettig oder verzweigt und enthält 1 bis 12, vorzugsweise 1 bis 10 und insbesondere 1 bis 5 Kohlenstoffatome. Beispielhaft seien Methyl, Ethyl, n- oder i-Propyl, n-, i- und t-Butyl und n-Pentyl genannt.

Als gegebenenfalls substituiertes Alkenyl steht geradkettiges oder verzweigtes Alkenyl mit vorzugsweise 2 bis 10 insbesondere 2 bis 7 Kohlenstoffatomen. Beispielhaft seien gegebenenfalls substituiertes Ethenyl, Propenyl-(1), Propenyl-(2), Butenyl-(1), Butenyl-(2) und Butenyl-(3) genannt.

Die Reste $R^3$ und $R^4$ können gemeinsam mit dem Atom, an welches sie gebunden sind, einen 3-bis 7-gliedrigen gesättigten Ring bilden, welcher durch 1 oder 2, vorzugsweise eine Alkylgruppe, insbesondere Methyl substituiert sein kann.

Als gegebenenfalls substituiertes Alkinyl steht geradkettiges oder verzweigtes Alkinyl mit vorzugsweise 2 bis 8, insbesondere 2 bis 5 Kohlenstoffatomen. Beispielhaft seien gegebenenfalls substituiertes Ethinyl, 2-Propinyl, 2-Butinyl, 3-Butinyl und 1-Methyl-2-propinyl genannt.

Die gegebenenfalls substituierten Reste $R^1$ bis $R^5$ können einen oder mehrere, vorzugsweise 1 bis 3, insbesondere 1 oder 2 gleiche oder verschiedene Substituenten tragen. Als Substituenten seien aufgeführt: Alkyl mit vorzugsweise 1 bis 10 insbesondere 1 bis 6 Kohlenstoffatomen, wie Methyl, Ethyl, n- und i-Propyl, n-, i- und t-Butyl.

Bevorzugt werden Verbindungen der allgemeinen Formel (I) als Repellentien verwendet, in welcher

- $R^1$ für Wasserstoff, $(C_1\text{-}C_{10})$-Alkyl, $(C_2\text{-}C_{10})$-Alkenyl oder $(C_2\text{-}C_{10})$-Alkinyl steht,
- $R^2$ und $R^5$ gleich oder verschieden sind und für $(C_1\text{-}C_{10})$-Alkyl, $(C_2\text{-}C_{10})$-Alkenyl oder $(C_2\text{-}C_{10})$-Alkinyl stehen und
- $R^3$ und $R^4$ für Wasserstoff, $(C_1\text{-}C_{10})$-Alkyl, jeweils im Arylteil gegebenenfalls einfach bis dreifach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten jeweils infrage kommen: Halogen, jeweils geradkettiges oder verzweigtes $(C_1\text{-}C_4)$-Alkyl, $(C_1\text{-}C_4)$-Halogenalkyl, $(C_1\text{-}C_4)$-Halogenalkoxy und $(C_1\text{-}C_4)$-Halogenalkylthio oder gemeinsam mit dem Atom, an welches sie gebunden sind, für Cycloalkyl mit 3 bis 7 Kohlenstoffatomen stehen.

Besonders bevorzugt werden Verbindungen der allgemeinen Formel (I) als Repellentien verwendet, in welcher

- $R^1$ für Wasserstoff, $(C_1\text{-}C_5)$-Alkyl, $(C_2\text{-}C_5)$-Alkenyl oder $(C_2\text{-}C_5)$-Alkinyl steht,
- $R^2$ für $(C_1\text{-}C_5)$-Alkyl, $(C_2\text{-}C_5)$-Alkenyl oder $(C_2\text{-}C_5)$-Alkinyl steht,
- $R^3$ und $R^4$ für Wasserstoff, $(C_1\text{-}C_5)$-Alkyl, jeweils im Phenylteil gegebenenfalls einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten jeweils infrage kommen: Fluor, Chlor, Brom, $(C_1\text{-}C_2)$-Alkyl, $(C_1\text{-}C_2)$-Halogenalkyl, $(C_1\text{-}C_2)$-Halogenalkoxy, $(C_1\text{-}C_2)$-Halogenalkylthio oder gemeinsam mit dem Atom, an welches sie gebunden sind, für Cycloalkyl mit 3 bis 6 Kohlenstoffatomen stehen und
- $R^5$ für $(C_1\text{-}C_{10})$-Alkyl, $(C_2\text{-}C_5)$-Alkenyl oder $(C_2\text{-}C_5)$-Alkinyl steht.

Ganz besonders bevorzugt werden die Verbindungen der Formel (I) verwendet, in welcher

- $R^1$ für Wasserstoff, $(C_1\text{-}C_5)$-Alkyl, $(C_2\text{-}C_5)$-Alkenyl oder $(C_2\text{-}C_5)$-Alkinyl steht,
- $R^2$ für $(C_1\text{-}C_5)$-Alkyl, $(C_2\text{-}C_5)$-Alkenyl oder $(C_2\text{-}C_5)$-Alkinyl steht,
- $R^3$ und $R^4$ für Wasserstoff, $(C_1\text{-}C_5)$-Alkyl, jeweils im Phenylteil gegebenenfalls einfach bis dreifach, gleich oder verschieden substituiertes Phenyl, Benzyl, Phenethyl, wobei als Substituenten jeweils infrage kommen: Fluor, Chlor, Brom, Methyl, Ethyl, Trifluormethyl, Trifluormethoxy, Trifluormethylthio oder
  gemeinsam mit dem Atom, an welches sie gebunden sind, für Cyclopropyl, Cyclopentyl, Cyclohexyl stehen und
- $R^5$ für $(C_1\text{-}C_{10})$-Alkyl, $(C_2\text{-}C_5)$-Alkenyl oder $(C_2\text{-}C_5)$-Alkinyl steht.

Weiterhin werden ganz besonders bevorzugt Verbindungen der Formel (I) verwendet, in welcher

- $R^1$ für Wasserstoff, $(C_1\text{-}C_5)$-Alkyl oder $(C_2\text{-}C_5)$-Alkenyl steht,
- $R^2$ für $(C_1\text{-}C_5)$-Alkyl oder $(C_2\text{-}C_5)$-Alkenyl steht,
- $R^3$ und $R^4$ für Wasserstoff, $(C_1\text{-}C_5)$-Alkyl oder gemeinsam mit dem Atom, an welches sie gebunden sind, für Cyclopropyl stehen und

$R^5$ für ($C_1$-$C_5$)-Alkyl oder ($C_2$-$C_5$)-Alkenyl steht.

Als Beispiele für die erfindungsgemäß zu verwendenden Verbindungen der Formel (I) seien N-Acetyl-2-butylaminoessigsäurebutylester und N-But-2-enyl-formamido-cyclopropancarbonsäureethylester genannt.

Diese Verbindungen werden wegen ihrer hervorragenden insekten- und milbenabweisenden Wirkung (Repellentwirkung) ganz besonders bevorzugt.

Außer den vorgenannten sowie den in den Herstellungsbeispielen aufgeführten Beispielen seien noch die folgenden genannt.

Allgemeine Formel

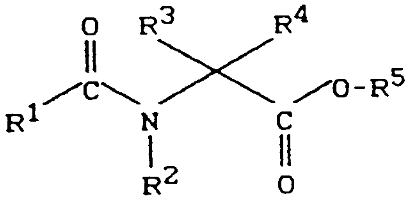

(I)

| $R^1$ | $R^2$ | $R^3$ | $R^4$ | $R^5$ |
|---|---|---|---|---|
| $CH_3$ | $n\text{-}C_4H_9$ | H | H | $C_2H_5$ |
| $CH_3$ | $n\text{-}C_4H_9$ | H | H | $n\text{-}C_4H_9$ |
| H | $n\text{-}C_4H_9$ | H | H | $n\text{-}C_4H_9$ |
| H | $n\text{-}C_4H_9$ | H | H | $C_2H_5$ |
| $n\text{-}C_4H_9$ | $CH_3$ | H | H | $C_2H_5$ |
| $n\text{-}C_4H_9$ | $CH_3$ | H | H | $n\text{-}C_4H_9$ |
| $CH_3$ | $n\text{-}C_4H_9$ | $CH_3$ | H | $C_2H_5$ |
| $CH_3$ | $n\text{-}C_4H_9$ | $CH_3$ | H | $n\text{-}C_4H_9$ |
| $n\text{-}C_4H_9$ | $CH_3$ | $CH_3$ | H | $C_2H_5$ |
| $n\text{-}C_4H_9$ | $CH_3$ | $CH_3$ | H | $n\text{-}C_4H_9$ |
| $C_2H_5$ | $n\text{-}C_4H_9$ | H | H | $C_2H_5$ |
| $n\text{-}C_4H_9$ | $C_2H_5$ | H | H | $n\text{-}C_4H_9$ |
| $C_2H_5$ | $n\text{-}C_4H_9$ | $CH_3$ | H | $C_2H_5$ |
| $C_2H_5$ | $n\text{-}C_4H_9$ | $CH_3$ | H | $n\text{-}C_4H_9$ |
| $n\text{-}C_4H_9$ | $C_2H_5$ | $CH_3$ | H | $C_2H_5$ |
| $n\text{-}C_4H_9$ | $C_2H_5$ | $CH_3$ | H | $n\text{-}C_4H_9$ |
| $CH_3$ | $n\text{-}C_4H_9$ | $C_2H_5$ | H | $C_2H_5$ |
| $C_2H_5$ | $n\text{-}C_4H_9$ | $C_2H_5$ | H | $n\text{-}C_4H_9$ |
| $n\text{-}C_4H_9$ | $CH_3$ | $C_2H_5$ | H | $C_2H_5$ |
| $n\text{-}C_4H_9$ | $CH_3$ | $C_2H_5$ | H | $n\text{-}C_4H_9$ |
| $n\text{-}C_4H_9$ | $C_2H_5$ | $C_2H_5$ | H | $C_2H_5$ |
| $n\text{-}C_4H_9$ | $C_2H_5$ | $C_2H_5$ | H | $n\text{-}C_4H_9$ |
| $CH_3$ | $n\text{-}C_4H_9$ | H | H | $CH(CH_3)C_2H_5$ |
| $CH_3$ | $CH_3$ | H | H | $CH(CH_3)C_2H_5$ |
| $CH(CH_3)C_2H_5$ | $CH_3$ | H | H | $C_2H_5$ |
| $CH(CH_3)C_2H_5$ | $CH_3$ | H | H | $n\text{-}C_4H_9$ |
| $CH_3$ | $CH_3$ | H | H | $t\text{-}C_4H_9$ |
| $CH_3$ | $CH(CH_3)C_2H_5$ | H | H | $C_2H_5$ |
| $CH_3$ | $CH(CH_3)C_2H_5$ | H | H | $CH_3$ |
| H | $CH(CH_3)C_2H_5$ | H | H | $n\text{-}C_4H_9$ |
| H | $CH_3$ | $-CH_2$ | $CH_2-$ | $CH(CH_3)C_2H_5$ |
| $CH_2CH{=}CH_2$ | $CH_3$ | H | H | $n\text{-}C_4H_9$ |
| $CH_3$ | $CH_2CH{=}CH_2$ | H | H | $n\text{-}C_4H_9$ |

Die erfindungsgemäß zu verwendenden acylierten α-Aminosäureester der Formel (I) sind teilweise bekannt und/oder können nach den an sich bekannten Verfahren hergestellt werden (vergl. EP-OS 136 615 und DE-OS 3 539 307).

Man erhält demgemäß die Verbindungen der Formel (I), wenn man

(A) die an sich bekannten oder nach bekannten Verfahren herstellbaren substituierten Amino-carbonsäureester (vergl. EP-OS 5782, EP-OS 25 141 und Cesare Ferri, Reaktionen der organischen Synthese,

Georg Thieme Verlag Stuttgart, 1978, S. 446-451) der Formel

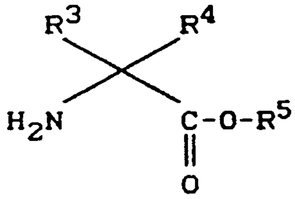

(II)

in welcher
$R^3$, $R^4$ und $R^5$ die oben angegebene Bedeutung haben,
zunächst mit an sich bekannten Carbonsäurechloriden der Formel (III)

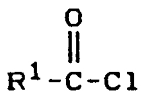

(III)

wobei
$R^1$ die unter Formel (I) angegebene Bedeutung hat, gegebenenfalls in Gegenwart eines Säureakzeptors, wie z.B. Triethylamin oder Kaliumcarbonat und gegebenenfalls unter Verwendung eines Verdünnungsmittels, wie z.B. Toluol, $CH_2Cl_2$, Tetrahydrofuran oder Acetonitril, bei Temperaturen zwischen -40 °C und 110 °C, umsetzt und dann
in einem zweiten Reaktionsschritt, gegebenenfalls nach Isolierung des Zwischenproduktes der Formel

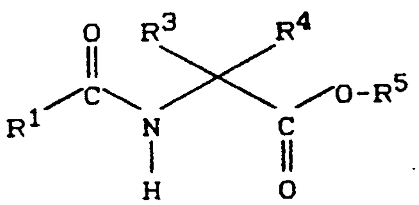

(IV)

in welcher
$R^1$, $R^3$, $R^4$ und $R^5$ die oben angegebene Bedeutung haben,
mit Halogeniden der Formel

$R^2$-X (V)

in welcher
$R^2$ die oben angegebene Bedeutung hat und
X für Chlor, Brom oder Iod, vorzugsweise für Brom und Iod steht,
gegebenenfalls in Gegenwart eines Säureakzeptors wie z.B. Triethylamin oder Kaliumcarbonat oder einer Base wie Natriumhydrid, gegebenenfalls in Gegenwart eines Phasentransferkatalysators, gegebenenfalls unter Verwendung eines Verdünnungsmittels, wie z.B. Toluol, Tetrahydrofuran oder Acetonitril, bei Temperaturen zwischen 0 und 110 °C umsetzt.
oder man erhält die Verbindungen der Formel (I), wenn man
(B) $\alpha$-Halogencarbonsäureester der Formel

$X\text{-}CH_2\text{-}COOR^5$ (VI)

in welcher
$R^5$ die oben angegebene Bedeutung hat und
X für Chlor, Brom oder Iod, vorzugsweise für Brom oder Iod steht,
mit Aminen der Formel

$R^2 - NH_2$ (VII)

in welcher

$R^2$ die oben angegebene Bedeutung hat,

gegebenenfalls in Gegenwart eines Säureakzeptors wie z.B. Triethylamin oder Kaliumcarbonat oder einer Base wie Natriumhydrid, gegebenenfalls unter Verwendung eines organischen Verdünnungsmittels, wie z.B. Toluol, Tetrahydrofuran oder Acetonitril vorzugsweise bei Temperaturen zwischen 0 und 110 °C umsetzt (vergl. z.B. Cesare Ferri, Reaktionen der organischen Synthese, Georg Thieme Verlag Stuttgart, 1978, S. 506-509) und dann in einem zweiten Reaktionsschritt, gegebenenfalls nach Isolierung des Zwischenproduktes der Formel

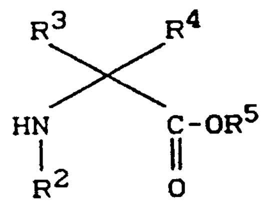

(VIII)

in welcher

$R^2$, $R^3$, $R^4$ und $R^5$ die oben angegebene Bedeutung haben,

mit an sich bekannten Carbonsäurechloriden der Formel

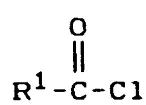

(III)

wobei

$R^1$ die unter Formel (I) angegebene Bedeutung hat,

gegebenenfalls in Gegenwart eines Säureakzeptors, wie z.B. Triethylamin oder Kaliumcarbonat und gegebenenfalls unter Verwendung eines Verdünnungsmittels, wie z.B. Toluol, $CH_2Cl_2$, Tetrahydrofuran oder Acetonitril, bei Temperaturen zwischen -40 °C und 110 °C, umsetzt.

Die Aufarbeitung gemäß Verfahrensvariante (A) und (B) erfolgt nach üblichen Methoden, beispielsweise durch Extraktion der Produkte mit Methylenchlorid oder Toluol aus der mit Wasser verdünnten Reaktionsmischung, Waschen der organischen Phase mit Wasser, Trocknen und Destillieren oder sogenanntes "Andestillieren", d.h. durch längeres Erhitzen unter vermindertem Druck auf mäßig erhöhte Temperaturen, um das Produkt von den letzten flüchtigen Bestandteilen zu befreien.

Eine weitere Reinigung kann durch Chromatographie an Kieselgel mit z.B. Hexan:Aceton = 7:3 als Laufmittel erfolgen.

Zur Charakterisierung der Verbindungen dienen Brechungsindex, Schmelzpunkt, Rf-Wert oder Siedepunkt.

Die gemäß Verfahrensvariante (A) und (B) zu verwendenden Verbindungen der Formel (III), (V), (VI) und (VII) sind allgemein bekannte Verbindungen der organischen Chemie.

Die Wirkung der Repellentien der allgemeinen Formel (I) hält lange an.

Sie können daher mit gutem Erfolg zur Abwehr von schädlichen oder lästigen, saugenden und beißenden Insekten und Milben verwendet werden.

Zu den saugenden Insekten gehören im wesentlichen die Stechmücken (z.B. Aedes-, Culex- und Anopheles-Arten), Schmetterlingsmücken (Phlebotomen), Gnitzen (Culicoides-Arten), Kriebelmücken (Simulium-Arten), Stechfliegen (z.B. Stomoxys calcitrans), Tsetse-Fliegen (Glossina-Arten), Bremsen (Tabanus-, Haematopota- und Chrysops-Arten), Stubenfliegen (z.B. Musca domestica und Fannia canicularis), Fleischfliegen (z.B. Sarcophaga carnaria), Myiasis erzeugende Fliegen (z.B. Lucilia cuprina, Chrysomyia chloropyga, Hypoderma bovis, Hypoderma lineatum, Dermatobia hominis, Oestrus ovis, Gasterophilus intestinalis, Cochliomyia hominovorax), Wanzen (z.B. Cimex lectularius, Rhodnius prolixus, Triatoma infestans), Läuse (z.B. Pediculus humanus, Haematopinus suis, Damalina oris), Lausfliegen (z.B. Melaphagus orinus), Flöhe (z.B. Pulex irritans), Cthenocephalides canis, Xenopsylla cheopis) und Sandflöhe (z.B. Dermatophilus penetrans).

Zu den beißenden Insekten gehören im wesentlichen Schaben (z.B. Blattella germanica, Periplaneta americana, Blatta orientalis, Supella supellectilium), Käfer (z.B. Sitophilus granarius, Tenebrio molitor,

Dermestes lardarius, Stegobium paniceum, Anobium puntactum, Hylotrupes bajulus), Termiten (z.B. Reticulitermes lucifugus) und Ameisen (z.B. Lasius niger).

Zu den Milben gehören Zecken (z.B. Ornithodorus moubata, Ixodes ricinus, Boophilus microplus, Amblyomma hebreum) und Milben in engerem Sinne (z.B. Sarcoptres scabiei, Dermanyssus gallinae).

Die erfindungsgemäß verwendbaren Wirkstoffe, die unverdünnt oder vorzugsweise verdünnt eingesetzt werden können, lassen sich in die für Repellents üblichen Formulierungen überführen. Sie lassen sich in allen in der Kosmetik üblichen Darreichungsformen einsetzen, beispielsweise in Form von Lösungen, Emulsionen, Gelen, Salben, Pasten, Cremes, Pulvern, Stiften, Sprays oder Aerosolen aus Sprühdosen.

Für die Anwendung im nichtkosmetischen Bereich lassen sich die Wirkstoffe z.B. in Granulate, Ölsprühmittel oder Slow-Release-Formulierungen einarbeiten.

Die Zubereitungen werden in bekannter Weise durch Vermischen oder Verdünnen der erfindungsgemäßen Wirkstoffe mit Lösungsmitteln (z.B. Xylol, Chlorbenzole, Paraffine, Methanol, Ethanol, Isopropanol, Wasser), Trägerstoffe (z.B. Kaoline, Tonerden, Talkum, Kreide, hochdisperse Kieselsäure, Silikate), Emulgiermitteln (z.B. Polyoxyethylen-Fettsäure-Ester,Polyoxyethylen-Fettalkohol-Ether, Alkylsulfonate, Arylsulfonate) und Dispergiermitteln (z.B. Lignin-Sulfitablaugen, Methylcellulose) hergestellt.

Die erfindungsgemäß verwendbaren Wirkstoffe können in den Formulierungen miteinander gemischt oder auch in Mischungen mit anderen bekannten Wirkstoffen (z.B. Sonnenschutzmittel) eingesetzt werden. Die Zubereitungen enthalten im allgemeinen zwischen 0,1 und 95 Gew.-% Wirkstoff, vorzugsweise zwischen 0,5 und 90 %.

Zum Schutz gegen blutsaugende Insekten oder Milben werden die erfindungsgemäßen Wirkstoffe entweder auf die menschliche oder tierische Haut aufgebracht oder Kleidungsstücke und andere Gegenstände damit behandelt.

Auch als Zusatz von Imprägniermitteln für beispielsweise Textilbahnen, Kleidungsstücke, Verpackungsmaterialien sowie als Zusatz zu Polier- , Putz- und Fensterreinigungsmitteln sind die erfindungsgemäß verwendbaren Wirkstoffe geeignet.

Die folgenden Beispiele für die Zubereitungen und die Verwendung der erfindungsgemäß verwendbaren Wirkstoffe dienen der weiteren Erläuterung der Erfindung.

Beispiel 1

Ein Repellentmittel in Form einer Lotion zur Anwendung auf der Haut wird hergestellt durch Vermischen von 30 Teilen eines der erfindungsgemäßen Wirkstoffe, 1,5 Teilen Parfüm und 68,5 Teilen Isopropanol. Isopropanol kann durch Ethanol ersetzt werden.

Beispiel 2

Ein Repellentmittel in Form eines Aerosols zum Aufsprühen auf die Haut wird hergestellt, indem man 50 % Wirkstofflösung, bestehend aus 30 Teilen eines der erfindungsgemäßen Wirkstoffe, 1,5 Teilen Parfüm, 68,5 Teilen Isopropanol, mit 50 % Frigen 11/12 (=halogenierter Kohlenwasserstoff als Treibgas) als Sprühdosenpräparation formuliert.

Beispiel 3

Eine andere Sprühdose setzt sich aus 40 % Wirkstofflösung, bestehend aus 20 Teilen eines der erfindungsgemäßen Wirkstoffe, 1 Teil Parfüm, 79 Teilen Isopropanol und 60 % Propan/Butan (Verhältnis 15:85) zusammen.

Es wurden individuelle Formulierungen entsprechend den Beispielen 1, 2 und 3 unter Einsatz folgender Wirkstoffe hergestellt: Verbindungen gemäß Herstellungsbeispielen Nr. 6 und 35.

Die folgenden Beispiele der biologischen Wirkung zeigen die Überlegenheit der erfindungsgemäßen Substanzen gegenüber dem Stand der Technik (Diethyltoluamid = DEET):

Beispiel A

Repellenttest am Meerschweinchen
Testtier: Aedes aegypti
Zahl der Testtiere: ca. 5000
Lösungsmittel: Ethanol (99,8 %)
3 Gewichtsteile Wirkstoff werden in 100 Volumenteilen Lösungsmittel aufgenommen.

Ein Meerschweinchen, dessen Rücken in einem Bereich von 50 $cm^2$ rasiert worden ist, wird in einem engen Käfig (Box) so fixiert, daß nur die rasierte Fläche den Mücken zugänglich ist. Nach Behandeln der Flache mit 0,4 ml Wirkstofflösung wird das Meerschweinchen nach Verdunsten des Lösungsmittels samt Box in einen 60 x 60 x 60 cm messenden Käfig gestellt, der nur mit Zuckerwasser gefütterte Testtiere beiderlei Geschlechts enthält.

Es wird für 10 Minuten beobachtet, wieviele Mücken das Meerschweinchen stechen.
Anschließend wird dieses herausgenommen und der Test nach einer Stunde wiederholt. Der Versuch wird maximal 9 Stunden lang durchgeführt oder so lange, bis die Wirkung abbricht.

Bei diesem Test zeigen z.B. die folgenden Verbindungen der Herstellungsbeispiele überlegene Wirkung gegenüber dem Stand der Technik (Diethyltoluamid = DEET).

**Beispiel A**

**Repellenttest am Meerschweinchen**

| Wirkstoff | Anzahl Einstiche nach: $0 - 6^h$ | $7 - 9^h$ |
|---|---|---|
| 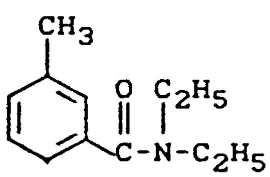 (bekannt) | 2,0 | 13,7 |
| 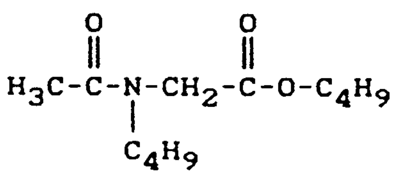 (6) | 0 | 0,7 |
| 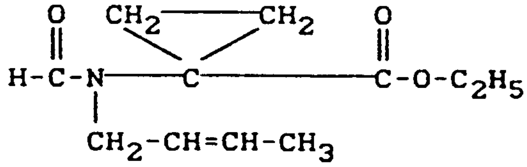 (35) | 1,3 | 4,7 |

Herstellungsbeispiele

Beispiel 1

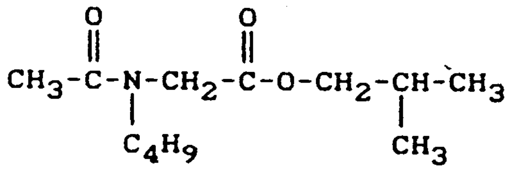

4,7 g (0,025 Mol) N-Butylaminoessigsäure-2-methylpropylester und 10 ml Triethylamin werden in 100 ml Tetrahydrofuran gelöst und bei 20 °C mit 5 ml Acetylchlorid versetzt. Man rührt 8 Stunden bei 20 °C, extrahiert mit Methylenchlorid/Wasser, trocknet und rotiert ein. Nach Filtration über Kieselgel (Laufmittel = Hexan:Aceton = 7:3) erhält man 5,3 g (ca. 92 % der Theorie) N-Acetyl-N-butyl-aminoessigsäure-2-methylpropylester mit einem Brechungsindex von $n_D^{20}$ = 1,4490.

Beispiel 2

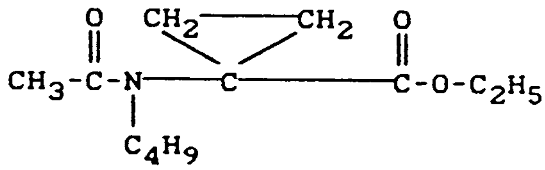

3,4 g (0,02 Mol) 1-Acetylamino-cyclopropan-1-carbonsäureethylester werden in 50 ml Tetrahydrofuran gelöst und mit 0,72 g (0,024 Mol) Natriumhydrid (80 %ig in Paraffin) versetzt, Man erhitzt 8 Stunden auf Rückfluß und gibt dann bei 20 °C 3,3 g n-Butylbromid (0,024 Mol) zur gut gerührten Lösung.

Man erwärmt 2 Stunden auf Rückflußtemperatur, kühlt ab und gibt 50 ml Ammoniumchlorid-Lösung zur Reaktionsmischung. Anschließend wird mit Methylenchlorid extrahiert, mit Magnesiumsulfat getrocknet, einrotiert und destilliert.

Man erhält 1,3 g N-Acetyl-N-butyl-1-amino-cyclopropan-1-carbonsäureethylester (ca. 29 % der Theorie) mit einem Siedepunkt von 117 °C/ 1 mbar.

In Analogie zu den vorgenannten Herstellungsbeispielen 1 und 2 wurden die in der folgenden Tabelle 1 aufgeführten Herstellungsbeispiele 3 bis 36 synthetisiert.

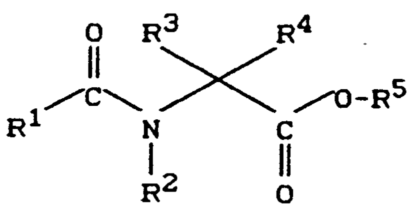

(I)

| Bsp. Nr. | $R^1$ | $R^2$ | $R^3$ | $R^4$ | $R^5$ | physikalische Daten |
|---|---|---|---|---|---|---|
| 3 | $C_2H_5$ | $C_4H_9$ | H | H | $C_2H_5$ | $n_D^{20}$ = 1,4500 |
| 4 | $C_4H_9$ | $CH_3$ | $-CH_2-CH_2-$ | | $C_2H_5$ | Kp 90-93° C/ 0,15 mbar |
| 5 | $CH_3$ | $C_4H_9$ | H | H | $C_3H_7$-i | $n_D^{20}$ = 1,4405 |
| 6 | $CH_3$ | $C_4H_9$ | H | H | $C_4H_9$ | $n_D^{20}$ = 1,4506 |
| 7 | $CH_3$ | $C_4H_9$ | H | H | $CH_2CHC_2H_5$<br>\|<br>$CH_3$ | $n_D^{20}$ = 1,4515 |
| 8 | $CH_3$ | $C_4H_9$ | H | H | $CH_2CH(C_2H_5)_2$ | $n_D^{20}$ = 1,4555 |
| 9 | $CH_3$ | $C_4H_9$ | H | H | $CHCH_2CH(CH_3)_2$<br>\|<br>$CH_3$ | $n_D^{20}$ = 1,4481 |
| 10 | $CH_3$ | $C_4H_9$ | H | H | $CH_2CH_2CH(CH_3)_2$ | $n_D^{20}$ = 1,4509 |
| 11 | $CH_3$ | $C_4H_9$ | H | H | $CH_2CH=CH_2$ | $n_D^{20}$ = 1,4625 |
| 12 | $CH_3$ | $C_4H_9$ | $CH_3$ | H | $C_4H_9$ | $n_D^{20}$ = 1,4507 |
| 13 | $CH_3$ | $C_4H_9$ | H | H | $C_2H_5$ | $n_D^{20}$ = 1,4502 |
| 14 | $C_4H_9$ | $CH_3$ | H | H | $C_4H_9$ | $n_D^{20}$ = 1,4514 |
| 15 | $CH_3$ | $CH_3$ | $-(CH_2)_5-$ | | $C_2H_5$ | $n_D^{20}$ = 1,4854 |
| 16 | $CH_3$ | $CH_3$ | $-(CH_2)_5-$ | | $C_4H_9$ | $n_D^{20}$ = 1,4811 |
| 17 | H | $CH_3$ | $-CH_2-CH_2-$ | | $CH_3$ | Kp: 80-90° C 0,2 mbar |
| 18 | H | $C_2H_5$ | $-CH_2-CH_2-$ | | $C_2H_5$ | $n_D^{20}$ : 1,4507 |
| 19 | H | $CH_3$ | $-CH_2-CH_2-$ | | $C_2H_5$ | $n_D^{20}$ : 1,4522 |
| 20 | H | $C_3H_7$ | $-CH_2-CH_2-$ | | $C_2H_5$ | $n_D^{20}$ : 1,4502 |

| Bsp. Nr. | $R^1$ | $R^2$ | $R^3$ $R^4$ | $R^5$ | physikalische Daten |
|---|---|---|---|---|---|
| 21 | H | $CH_3$ | $-CH_2-CH_2-$ | $C_3H_7$ | $n_D^{20}$ =1,4510 |
| 22 | H | $CH_3$ | $-CH_2-CH_2-$ | $C_4H_9$ | $n_D^{20}$ =1,4475 |
| 23 | H | $CH_3$ | $-CH_2-CH_2-$ | $C_4-H_9-i$ | $n_D^{20}$ =1,4481 |
| 24 | H | $CH_3$ | $-CH_2-CH_2-$ | $C_5H_{11}$ | |
| 25 | H | $CH_3$ | $-CH_2-CH_2-$ | $CH_2-C_5H_{11}$ | Kp110- 105° C /0,07mbar |
| 26 | H | $CH_3$ | $-CH_2-CH_2-$ | $CH_2-C_7H_{15}$ | Kp110- 105° C /0,2 mbar |
| 27 | H | $CH_3$ | $-CH_2-CH_2-$ | $CH_2-CH-C_2H_5$<br>\|<br>$C_2H_5$ | Kp102- 105° C /0,05 mbar |
| 28 | H | $CH_3$ | $-CH_2-CH_2-$ | $C_4H_9-t$ | $n_D^{20}$ =1,4478 |
| 29 | H | $CH_3$ | $-CH_2-CH_2-$ | $C_{10}H_{21}$ | Kp110- 120° C /0,02 mbar |
| 30 | H | $CH_3$ | $-CH_2-CH_2-$ | $CH_2-CH_2$<br>\|<br>$CH(CH_3)_2$ | Kp 80-90/ 0,4 mbar |
| 31 | H | $CH_3$ | $-CH_2-CH_2-$ | $CH-CH-C_4H_9$<br>\|<br>$C_2H_5$ | Kp 90-100° C 0,1 mbar |
| 32 | H | $CH_3$ | $-CH_2-CH_2-$ | $C_9H_{19}$ | Kp105-110° C /0,2 mbar |
| 33 | H | $C_4H_9$ | $-CH_2-CH_2-$ | $C_2H_5$ | Kp 80-90° C/ 0,2 mbar |
| 34 | H | $CH_2-CH=CH_2$ | $-CH_2-CH_2-$ | $C_2H_5$ | $n_D^{20}$ =1,4702 |
| 35 | H | $CH_2-CH=CH$<br>\|<br>$CH_3$ | $-CH_2-CH_2-$ | $C_2H_5$ | Kp 95-100° C /0,2 mbar |
| 36 | H | $C_5H_{11}$ | $-CH_2-CH_2-$ | $C_2H_5$ | $n_D^{23}$ =1,4496 |

Herstellung der Vorprodukte:

N-Butylaminoessigsäure-2-methylpropylester

20 g (0,27 Mol) n-Butylamin werden in 100 ml Acetonitril gelöst und bei 20 ° C mit 7,8 g (0,04 Mol) Bromessigsäure-2-methyl-propylester versetzt. Man rührt 2 Stunden bei 20 ° C, rotiert ein, nimmt in

Methylenchlorid/Wasser auf, trocknet die organische Phase, rotiert ein und filtriert über Kieselgel (Laufmittel Hexan:Aceton = 7:3).

Man erhält 5,3 g (71 % der Theorie) N-Butylaminoessigsäure-2-methyl-propylester.

Bromessigsäure-2-methyl-propylester

41 g (0,2 Mol) Bromacetylbromid werden in 400 ml Tetrahydrofuran gelöst und bei 20 °C mit einer Mischung aus 36 ml Triethylamin und 15 g sec-Butanol, gelöst in 20 ml Tetrahydrofuran, versetzt. Man rührt 8 Stunden bei 20 °C und extrahiert mit Wasser/Methylenchlorid. Nach Trocknen, Einrotieren und Destillieren erhält man 13,9 g (36 % der Theorie) Bromessigsäure-2-methyl-propylester.

1-Acetylamino-cyclopropancarbonsäureethylester

96,2 g (0,7 Mol) 1-Amino-cyclopropancarbonsäurehydrochlorid werden in 700 ml Ethanol gelöst und unter Einleiten von Chlorwasserstoff auf Rückfluß erhitzt. Nach 2,5 Stunden wird abgekühlt, einrotiert, mit Wasser aufgenommen, mit Natriumhydrogencarbonat auf pH 8-9 gestellt und mit Methylenchlorid extrahiert. Die Organische Phase wird getrocknet, einrotiert und destilliert.

Man erhält 39 g (43 % der Theorie) 1-Amino-cyclopropancarbonsäureethylester mit einem Siedepunkt von 33 °C/0,3 mbar.

3,9 g (0,03 Mol) dieser Verbindung werden mit 6 ml Triethylamin und 50 ml Tetrahydrofuran gemischt und bei 20 °C mit 3,2 ml Acetylchlorid versetzt. Man rührt 8 Stunden bei 20 °C, extrahiert mit Methylenchlorid/Wasser, trocknet die organische Phase, rotiert ein und destilliert mit einem Kugelrohrofen.

Man erhält 4,1 g (80 % der Theorie) 1-Acetylamino-cyclopropancarbonsäureethylester von Siedepunkt 150 °C/0,2 mbar.

## Patentansprüche

**1.** Verwendung von acylierten α-Aminosäureester-Derivaten der allgemeinen Formel (I)

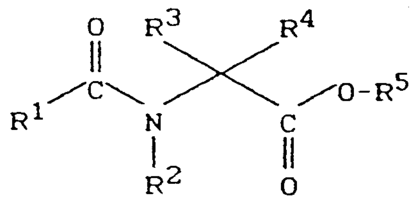

(I)

zur Insekten- und Milbenabwehr, wobei in Formel (I)

- $R^1$ für Wasserstoff oder für einen gegebenenfalls substituierten Alkyl-, Alkenyl- oder Alkinylrest steht,
- $R^2$ und $R^5$ gleich oder verschieden sind und für gegebenenfalls substituierte Alkyl-, Alkenyl- oder Alkinylreste stehen und
- $R^3$ und $R^4$ für Wasserstoff, gegebenenfalls substituiertes Alkyl, Aralkyl oder Aryl stehen oder gemeinsam mit dem Atom, an welches sie gebunden sind, einen gegebenenfalls substituierten, monocyclischen Ring bilden.

**2.** Verwendung von acylierten α-Aminosäureester-Derivaten der Formel (I) gemäß Anspruch 1 zur Insekten- und Milbenanwehr dadurch gekennzeichnet, daß in Formel (I)

- $R^1$ für Wasserstoff, $(C_1\text{-}C_{10})$-Alkyl, $(C_2\text{-}C_{10})$-Alkenyl oder $(C_2\text{-}C_{10})$-Alkinyl steht,
- $R^2$ und $R^5$ gleich oder verschieden sind und für $(C_1\text{-}C_{10})$-Alkyl, $(C_2\text{-}C_{10})$-Alkenyl oder $(C_2\text{-}C_{10})$-Alkinyl stehen und
- $R^3$ und $R^4$ für Wasserstoff, $(C_1\text{-}C_{10})$-Alkyl, jeweils im Arylteil gegebenenfalls einfach bis dreifach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten jeweils infrage kommen: Halogen, jeweils geradkettiges oder verzweigtes $(C_1\text{-}C_4)$-Alkyl, $(C_1\text{-}C_4)$-Halogenalkyl, $(C_1\text{-}C_4)$-Halogenalkoxy und $(C_1\text{-}C_4)$-Halogenalkylthio oder gemeinsam mit dem Atom, an welches sie gebunden sind, für Cycloalkyl mit 3 bis 7 Kohlenstoffatomen stehen.

3. Verwendung von acylierten α-Aminosäureester-Derivaten der Formel (I) gemäß Ansprch 1 zur Insekten- und Milbenabwehr, dadurch gekennzeichnet, daß in Formel (I)
   - $R^1$ für Wasserstoff, ($C_1$-$C_5$)-Alkyl, ($C_2$-$C_5$)-Alkenyl oder ($C_2$-$C_5$)-Alkinyl steht,
   - $R^2$ für ($C_1$-$C_5$)-Alkyl, ($C_2$-$C_5$)-Alkenyl oder ($C_2$-$C_5$)-Alkinyl steht,
   - $R^3$ und $R^4$ für Wasserstoff, ($C_1$-$C_5$)-Alkyl, jeweils im Phenylteil gegebenenfalls einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten jeweils infrage kommen: Fluor, Chlor, Brom, ($C_1$-$C_2$)-Alkyl, ($C_1$-$C_2$)-Halogenalkyl, ($C_1$-$C_2$)-Halogenalkoxy, ($C_1$-$C_2$)-Halogenalkylthio oder gemeinsam mit dem Atom, an welches sie gebunden sind, für Cycloalkyl mit 3 bis 6 Kohlenstoffatomen stehen und
   - $R^5$ für ($C_1$-$C_{10}$)-Alkyl, ($C_2$-$C_5$)-Alkenyl oder ($C_2$-$C_5$)-Alkinyl steht.

4. Verwendung von acylierten α-Aminosäureester-Derivaten der Formel (I) gemäß Anspruch 1 zur Insekten- und Milbenabwehr, dadurch gekennzeichnet, daß in Formel (I)
   - $R^1$ für Wasserstoff, ($C_1$-$C_5$)-Alkyl, ($C_2$-$C_5$)-Alkenyl oder ($C_2$-$C_5$)-Alkinyl steht,
   - $R^2$ für ($C_1$-$C_5$)-Alkyl, ($C_2$-$C_5$)-Alkenyl oder ($C_2$-$C_5$)-Alkinyl steht,
   - $R^3$ und $R^4$ für Wasserstoff, ($C_1$-$C_5$)-Alkyl, jeweils im Phenylteil gegebenenfalls einfach bis dreifach, gleich oder verschieden substituiertes Phenyl, Benzyl, Phenethyl, wobei als Substituenten jeweils infrage kommen: Fluor, Chlor, Brom, Methyl, Ethyl, Trifluormethyl, Trifluormethoxy, Trifluormethylthio oder
     gemeinsam mit dem Atom, an welches sie gebunden sind, für Cyclopropyl, Cyclopentyl, Cyclohexyl stehen und
   - $R^5$ für ($C_1$-$C_{10}$)-Alkyl, ($C_2$-$C_5$)-Alkenyl oder ($C_2$-$C_5$)-Alkinyl steht.

5. Verwendung von acylierten α-Aminosäureester-Derivaten der Formel (I) gemäß Anspruch 1 zur Insekten- und Milbenabwehr, dadurch gekennzeichnet, daß in der Formel (I)
   - $R^1$ für Wasserstoff, ($C_1$-$C_5$)-Alkyl oder ($C_2$-$C_5$)-Alkenyl steht,
   - $R^2$ für ($C_1$-$C_5$)-Alkyl oder ($C_2$-$C_5$)-Alkenyl steht,
   - $R^3$ und $R^4$ für Wasserstoff, ($C_1$-$C_5$)-Alkyl oder gemeinsam mit dem Atom, an welches sie gebunden sind, für Cyclopropyl stehen und
   - $R^5$ für ($C_1$-$C_5$)-Alkyl oder ($C_2$-$C_5$)-Alkenyl steht.

6. Verfahren zur Insekten- und Milbenabwehr, dadurch gekennzeichnet, daß man acylierte α-Aminosäureester-Derivate der allgemeinen Formel (I) einsetzt.

## Claims

1. Use of acylated α-amino acid ester derivatives of the general formula (I)

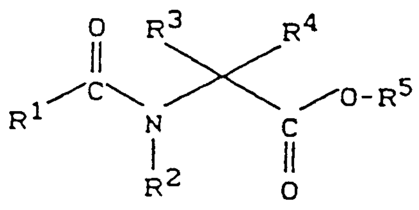

(I)

for repulsing insects and mites, where in formula (I)
   - $R^1$ represents hydrogen or an optionally substituted alkyl, alkenyl or alkinyl radical,
   - $R^2$ and $R^5$ are identical or different and represent optionally substituted alkyl, alkenyl or alkinyl radicals and
   - $R^3$ and $R^4$ represent hydrogen or optionally substituted alkyl, aralkyl or aryl, or together with the atom to which they are bonded form an optionally substituted, monocyclic ring.

2. Use of acylated α-amino acid ester derivatives of the formula (I) according to Claim 1 for repulsing insects and mites, characterised in that, in formula (I),
   - $R^1$ represents hydrogen, ($C_1$-$C_{10}$)-alkyl, ($C_2$-$C_{10}$)-alkenyl or $C_2$-$C_{10}$)-alkinyl,
   - $R^2$ and $R^5$ are identical or different and represent ($C_1$-$C_{10}$)-alkyl, ($C_2$-$C_{10}$)-alkenyl or ($C_2$-$C_{10}$)-

alkinyl and

$R^3$ and $R^4$ represent hydrogen, $(C_1-C_{10})$-alkyl, aryl or aralkyl, each of which is optionally substituted in the aryl moiety by one to three identical or different substituents, and each of which has 6 to 10 carbon atoms in the aryl moiety and if appropriate 1 to 4 carbon atoms in the alkyl moiety, suitable aryl substituents in each case being: halogen, in each case straight-chain or branched $(C_1-C_4)$-alkyl, $(C_1-C_4)$-halogenoalkyl, $(C_1-C_4)$-halogenoalkoxy and $(C_1-C_4)$-halogenoalkylthio, or, together with the atom to which they are bonded, represent cycloalkyl having 3 to 7 carbon atoms.

**3.** Use of acylated $\alpha$-amino acid ester derivatives of the formula (I) according to Claim 1 for repulsing insects and mites, characterised in that, in formula (I)

$R^1$ represents hydrogen, $(C_1-C_5)$-alkyl, $(C_2-C_5)$-alkenyl or $(C_2-C_5)$-alkinyl,

$R^2$ represents $(C_1-C_5)$-alkyl, $(C_2-C_5)$-alkyl or $(C_2-C_5)$-alkinyl,

$R^3$ and $R^4$ represent hydrogen, $(C_1-C_5)$-alkyl, phenyl or phenylalkyl which have, if appropriate, 1 or 2 carbon atoms in the alkyl moiety and each of which is optionally substituted by one to three identical or different substituents, suitable phenyl substituents in each case being: fluorine, chlorine, bromine, $(C_1-C_2)$-alkyl, $(C_1-C_2)$-halogenoalkyl, $(C_1-C_2)$-halogenoalkoxy, $(C_1-C_2)$-halogenoalkylthio or, together with the atom to which they are bonded, represent cycloalkyl having 3 to 6 carbon atoms and

$R^5$ represents $(C_1-C_{10})$-alkyl, $(C_2-C_5)$-alkenyl or $(C_2-C_5)$-alkinyl.

**4.** Use of acylated $\alpha$-amino acid ester derivatives of the formula (I) according to Claim 1 for repulsing insects and mites, characterised in that, in formula (I)

$R^1$ represents hydrogen, $(C_1-C_5)$-alkyl, $(C_2-C_5)$-alkenyl or $(C_2-C_5)$-alkinyl,

$R^2$ represents $(C_1-C_5)$-alkyl, $(C_2-C_5)$-alkenyl or $(C_2-C_5)$-alkinyl,

$R^3$ and $R^4$ represent hydrogen or $(C_1-C_5)$-alkyl, or phenyl, benzyl or phenethyl, each of which is optionally substituted in the phenyl moiety by one to three identical or different substituents, suitable substituents in each case being: fluorine, chlorine, bromine, methyl, ethyl, trifluoromethyl, trifluoromethoxy, trifluoromethylthio or together with the atom to which they are bonded represent cyclopropyl, cyclopentyl or cyclohexyl and

$R^5$ represents $(C_1-C_{10})$-alkyl, $(C_2-C_5)$-alkenyl or $(C_2-C_5)$-alkinyl.

**5.** Use of acylated $\alpha$-amino acid ester derivatives of the formula (I) according to Claim 1 for repulsing insects and mites, characterised in that, in formula (I)

$R^1$ represents hydrogen, $(C_1-C_5)$-alkyl or $(C_2-C_5)$-alkenyl

$R^2$ represents $(C_1-C_5)$-alkyl or $(C_2-C_5)$-alkenyl,

$R^3$ and $R^4$ represent hydrogen, $(C_1-C_5)$-alkyl or together with the atom to which they are bonded represent cyclopropyl and

$R^5$ represents $(C_1-C_5)$-alkyl or $(C_2-C_5)$-alkenyl.

**6.** Method for repulsing insects and mites, characterised in that acylated $\alpha$-amino acid ester derivatives of the general formula (I) are employed.

## Revendications

**1.** Utilisation pour la lutte contre les insectes et les acariens de dérivés d'esters d'acides $\alpha$-aminés acylés de formule générale (I)

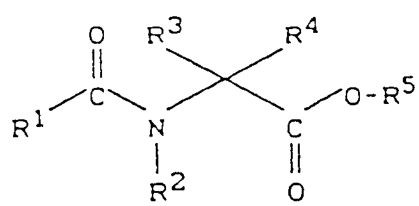

(I)

dans laquelle

$R^1$ représente l'hydrogène ou un reste alkyle, alcényle ou alcynyle éventuellement substitué,

$R^2$ et$R^5$ sont identiques ou différents et représentent des restes alkyle, alcényle ou alcynyle éventuellement substitués et
$R^3$ et $R^4$ représentent l'hydrogène, un reste alkyle, aralkyle ou aryle éventuellement substitué ou bien forment avec l'atome auquel ils sont liés un noyau monocyclique éventuellement substitué.

**2.** Utilisation de dérivés d'esters d'acides $\alpha$-aminés acylés de formule (I) selon la revendication 1 pour la lutte contre les insectes et les acariens, caractérisée en ce que, dans la formule (I)
$R^1$ représente l'hydrogène, un groupe alkyle en $C_1$-$C_{10}$, alcényle en $C_2$-$C_{10}$ ou alcynyle en $C_2$-$C_{10}$,
$R^2$ et $R^5$ sont identiques ou différents et représentent des groupes alkyle en $C_1$-$C_{10}$, alcényle en $C_2$-$C_{10}$ ou alcynyle en $C_2$-$C_{10}$ et
$R^3$ et $R^4$ représentent l'hydrogène, un groupe alkyle en $C_1$-$C_{10}$, un groupe aryle ou aralkyle contenant 6 à 10 atomes de carbone dans la partie aryle et éventuellement 1 à 4 atomes de carbone dans la partie alkyle qui est éventuellement substitué une à trois fois de manière identique ou différente, les substituants de la partie aryle pouvant être : un halogène, un groupe alkyle en $C_1$-$C_4$, halogénoalkyle en $C_1$-$C_4$, halogénoalcoxy en $C_1$-$C_4$ ou halogénoalkylthio en $C_1$-$C_4$ linéaire ou ramifié, ou bien forment avec l'atome auquel ils sont liés un groupe cycloalkyle ayant 3 à 7 atomes de carbone.

**3.** Utilisation de dérivés d'esters d'acides $\alpha$-aminés acylés de formule (I) selon la revendication 1 pour la lutte contre les insectes et les acariens, caractérisée en ce que, dans la formule (I)
$R^1$ représente l'hydrogène, un groupe alkyle en $C_1$-$C_5$, alcényle en $C_2$-$C_5$ ou alcynyle en $C_2$-$C_5$,
$R^2$ représente un groupe alkyle en $C_2$-$C_5$, alcényle en $C_2$-$C_5$ ou alcynyle en $C_2$-$C_5$,
$R^3$ et $R^4$ représentent l'hydrogène, un groupe alkyle en $C_1$-$C_5$, un groupe phényle ou phénylalkyle ayant éventuellement 1 ou 2 atomes de carbone dans la partie alkyle et éventuellement substitué une à trois fois de manière identique ou différente dans la partie phényle, les substituants de la partie phényle pouvant être le fluor, le chlore, le brome, un groupe alkyle en $C_1$-$C_2$, halogénoalkyle en $C_1$-$C_2$, halogénoalcoxy en $C_1$-$C_2$, halogénoalkylthio en $C_1$-$C_2$, ou bien forment avec l'atome auquel ils sont liés un groupe cycloalkyle ayant 3 à 6 atomes de carbone, et $R^5$ représente un groupe alkyle en $C_1$-$C_{10}$, alcényle en $C_2$-$C_5$ ou alcynyle en $C_2$-$C_5$.

**4.** Utilisation de dérivés d'esters d'acides $\alpha$-aminés acylés de formule (I) selon la revendication 1 pour la lutte contre les insectes et les acariens, caractérisée en ce que, dans la formule (I)
$R^1$ représente l'hydrogène, un groupe alkyle en $C_1$-$C_5$, alcényle en $C_2$-$C_5$ ou alcynyle en $C_2$-$C_5$,
$R^2$ représente un groupe alkyle en $C_1$-$C_5$, alcényle en $C_2$-$C_5$ ou alcynyle en $C_2$-$C_5$,
$R^3$ et $R^4$ représentent l'hydrogène, un groupe alkyle en $C_1$-$C_5$, un groupe phényle, benzyle, phénéthyle éventuellement substitué une à trois fois de manière identique ou différente dans la partie phényle, les substituants pouvant être le fluor, le chlore, le brome, un groupe méthyle, éthyle, trifluorométhyle, trifluorométhoxy, trifluorométhylthio, ou bien forment avec l'atome auquel ils sont liés un groupe cyclopropyle, cyclopentyle, cyclohexyle, et
$R^5$ représente un groupe alkyle en $C_1$-$C_{10}$, alcényle en $C_2$-$C_5$ ou alcynyle en $C_2$-$C_5$.

**5.** Utilisation de dérivés d'esters d'acides $\alpha$-aminés acylés de formule (I) selon la revendication 1 pour la lutte contre les insectes et les acariens, caractérisée en ce que, dans la formule (I)
$R^1$ représente l'hydrogène, un groupe alkyle en $C_1$-$C_5$ ou alcényle en $C_2$-$C_5$,
$R^2$ représente un groupe alkyle en $C_1$-$C_5$ ou alcényle en $C_2$-$C_5$,
$R^3$ et $R^4$ représentent l'hydrogène, un groupe alkyle en $C_1$-$C_5$ ou forment avec l'atome auquel ils sont liés un groupe cyclopropyle, et
$R^5$ représente un groupe alkyle en $C_1$-$C_5$ ou alcényle en $C_2$-$C_5$.

**6.** Procédé de lutte contre les insectes et les acariens, caractérisé en ce que l'on utilise des dérivés d'esters d'acides $\alpha$-aminés acylés de formule générale (I).